# EUROPEAN PATENT APPLICATION

(11) **EP 1 538 159 A1**
(43) Date of publication of application: **08.06.2005**
(21) Application number: 03028120.8
(22) Date of filing: 05.12.2003
(51) Int. Cl.: C07K 5/078, C07K 5/065, C07D 401/12, C07D 207/26, A61K 31/496, A61P 3/04

(54) **Substituted N-benzyl-lactam derivatives as melanocortin-4 receptor agonists**

(71) Applicant: Santhera Pharmaceuticals (Schweiz) GmbH, 4410 Liestal (CH)
(72) Inventor: Soeberdt, Michael, 79618 Rheinfelden (DE); Weyermann, Philipp, 4450 Sissach (CH); von Sprecher, Andreas, 4104 Oberwil (CH)
(74) Representative: Grünecker, Kinkeldey, Stockmair & Schwanhäusser Anwaltssozietät

(57) **Abstract**

The present invention relates to novel substituted N-benzyl-lactam derivatives as melanocortin-4 receptor (MC-4R) agonists. The MC-4R agonists of the invention can be used for the treatment of disorders and diseases such as obesity, diabetes, and sexual dysfunction. All diseases and disorders where the activation of the MC-4R is involved can be treated with the compounds of the invention.

## Description

### Field of the Invention

The present invention relates to novel substituted N-benzyl-lactam derivatives with improved biological activity as melanocortin-4 receptor agonists especially in comparison with the exemplified compounds of the patent applications WO03009847A1 and WO03009850A1. The compounds of the invention are selective agonists of the human melanocortin-4 receptor (MC-4R). The agonists can be used for the treatment of disorders and diseases such as obesity, diabetes and sexual dysfunction. Generally all diseases and disorders where the activation of the MC-4R is involved can be treated with the compounds of the invention.

### Background of the Invention

Melanocortins (MCs) stem from pro-opiomelanocortin (POMC) via proteolytic cleavage. These peptides, adrenocorticotropic hormone (ACTH), α-melanocyte-stimulating hormone (α-MSH), β-MSH and γ-MSH, range in size from 12 to 39 amino acids. The most important endogenous agonist for central MC-4R activation appears to be the tridecapeptide α-MSH. Among MCs, it was reported that α-MSH acts as a neurotransmitter or neuromodulator in the brain. MC peptides, particularly α-MSH, have a wide range of effects on biological functions including feeding behavior, pigmentation, and exocrine function. The biological effects of α-MSH are mediated by a sub-family of 7-transmembrane G-protein-coupled receptors, termed melanocortin receptors (MC-Rs). Activation of any of these MC-Rs results in stimulation of cAMP formation.

To date, five distinct types of receptor subtype for MC (MC-1 R to MC-5R) have been identified, and these are expressed in different tissues.

MC-1R was first found in melanocytes. Naturally occurring inactive variants of MC-1R in animals were shown to lead to alterations in pigmentation and a subsequent lighter coat color by controlling the conversion of phaeomelanin to eumelanin through the control of tyrosinase. From these, and other studies, it is evident that MC-1 R is an important regulator of melanin production and coat color in animals and skin color in humans.

The MC-2R is expressed in the adrenal gland representing the ACTH receptor. The MC-2R is not a receptor for α-MSH but is the receptor for the adrenocorticotropic hormone I (ACTH I).

The MC-3R is expressed in the brain (predominately located in the hypothalamus) and peripheral tissues like gut and placenta, and knock-out studies have revealed that the MC-3R may be responsible for alterations in feeding behavior, body weight and thermogenesis.

The MC-4R is primarily expressed in the brain. Overwhelming data support the role of MC-4R in energy homeostasis. Genetic knock-outs and pharmacologic manipulation of MC-4R in animals have shown that agonizing the MC-4R causes weight loss and antagonizing the MC-4R produces weight gain. (A. Kask, et al., "Selective antagonist for the melanocortin-4 receptor (HS014) increases food intake in free-feeding rats," Biochem. Biophys. Res. Commun., 245: 90-93 (1998)).

MC-5R is ubiquitously expressed in many peripheral tissues including white fat, placenta and a low level of expression is also observed in the brain. However its expression is greatest in exocrine glands. Genetic knock-out of this receptor in mice results in altered regulation of exocrine gland function, leading to changes in water repulsion and thermoregulation. MC-5R knockout mice also reveal reduced sebaceous gland lipid production (Chen et al., Cell, 91: 789-798 (1997)).

Attention has been focused on the study of MC-3R and MC-4R modulators and their use in treating body weight disorders, such as obesity and anorexia. However, evidence has shown that the MC peptides have potent physiological effects besides their role in regulating pigmentation, feeding behavior and exocrine function. In particular, α-MSH recently has been shown to induce a potent anti-inflammatory effect in both acute and chronic models of inflammation including inflammatory bowel-disease, renal ischemia/reperfusion injury and endotoxin-induced hepatitis. Administration of α-MSH in these models results in substantial reduction of inflammation-mediated tissue damage, a significant decrease in leukocyte infiltration, and a dramatic reduction in elevated levels of cytokines and other mediators to near baseline levels. Recent studies have demonstrated that the anti-inflammatory actions of α-MSH are mediated by MC-1 R. The mechanism by which agonism of MC-1 R results in an anti-inflammatory response is likely through inhibition of the pro-inflammatory transcription activator, NF-κB. NF-κB is a pivotal component of the pro-inflammatory cascade, and its activation is a central event in initiating many inflammatory diseases. Additionally, anti-inflammatory actions of α-MSH may be in part mediated by agonism of MC-3R and/or MC-5R.

A specific single MC-R that may be targeted for the control of obesity has not yet been identified, although evidence has been presented that MC-4R signaling is important in mediating feeding behavior (S.Q. Giraudo et al., "Feeding effects of hypothalamic injection of melanocortin-4 receptor ligands", Brain Research, 80: 302-306 (1998)). Further evidence for the involvement of MC-Rs in obesity includes: a) the agouti (A^{vy}) mouse which ectopically expresses an antagonist of the MC-1 R, MC-3R and MC-4R is obese, indicating that blocking the action of these three MC-Rs can lead to hyperphagia and metabolic disorders; 2) MC-4R knockout mice (D. Huszar et al., Cell, 88: 131-141 (1997)) recapitulate the phenotype of the agouti mouse and these mice are obese; 3) the cyclic heptapeptide melanotanin II (MT-II) (a non-selective MC-1 R, -3R, -4R and -5R agonist) injected intracerebroventricularly (ICV) in rodents, reduces food intake in several animal feeding models (NPY, ob/ob, agouti, fasted), while ICV injected SHU-9119 (MC-3R and -4R antagonist; MC-1 R, and -5R agonist) reverses this effect and can induce hyperphagia; 4) chronic intraperitoneal treatment of Zucker fatty rats with an α-NDP-MSH derivative (HP-228) has been reported to activate MC1-R, -3R, -4R and -5R and to attenuate food intake and body weight gain over a 12 week period (I. Corcos et al., "HP-228 is a potent agonist of melanocortin receptor-4 and significantly attenuates obesity and diabetes in Zucker fatty rats", Society for Neuroscience Abstracts, 23: 673 (1997)).

MC-4R appears to play a role in other physiological functions as well, namely controlling grooming behavior, erection and blood pressure. Erectile dysfunction denotes the medical condition of inability to achieve penile erection sufficient for successful intercourse. The term "impotence" is often employed to describe this prevalent condition. Synthetic melanocortin receptor agonists have been found to initiate erections in men with psychogenic erectile dysfunction (H. Wessells et al., "Synthetic Melanotropic Peptide Initiates Erections in Men With Psychogenic Erectile Dysfunction: Double-Blind, Placebo Controlled Crossover Study", J. Urol., 160: 389-393, 1998). Activation of melanocortin receptors of the brain appears to cause normal stimulation of sexual arousal. Evidence for the involvement of MC-R in male and/or female sexual dysfunction is detailed in WO/0074679.

Diabetes is a disease in which a mammal's ability to regulate glucose levels in the blood is impaired because the mammal has a reduced ability to convert glucose to glycogen for storage in muscle and liver cells. In Type I diabetes, this reduced ability to store glucose is caused by reduced insulin production. "Type II diabetes" or "Non-Insulin Dependent Diabetes Mellitus" (NIDDM) is the form of diabetes, which is due to a profound resistance to insulin stimulating or regulatory effect on glucose and lipid metabolism in the main insulin-sensitive tissues, muscle, liver and adipose tissue. This resistance to insulin responsiveness results in insufficient insulin activation of glucose uptake, oxidation and storage in muscle and inadequate insulin repression of lipolysis in adipose tissue and of glucose production and secretion in liver. When these cells become desensitized to insulin, the body tries to compensate by producing abnormally high levels of insulin and hyperinsulemia results. Hyperinsulemia is associated with hypertension and elevated body weight. Since insulin is involved in promoting the cellular uptake of glucose, amino acids and triglycerides from the blood by insulin sensitive cells, insulin insensitivity can result in elevated levels of triglycerides and LDL which are risk factors in cardiovascular diseases. The constellation of symptoms which includes hyperinsulemia combined with hypertension, elevated body weight, elevated triglycerides and elevated LDL is known as Syndrome X. MC-4R agonists might be useful in the treatment of NIDDM and Syndrome X.

Among MC receptor subtypes, the MC4 receptor is also of interest in terms of the relationship to stress and the regulation of emotional behavior, as based on the following findings. Stress initiates a complex cascade of responses that include endocrine, biochemical and behavioral events. Many of these responses are initiated by release of corticotropin-releasing factor (CRF), (Owen MJ and Nemeroff CB (1991). Physiology and pharmacology of corticotrophin releasing factor. *Pharmacol Rev* 43: 425 - 473). In addition to activation of the brain CRF system, there are several lines of evidence that melanocortins (MCs), which stem from proopiomelanocortin by enzymatic processing, mediate important behavioral and biochemical responses to stress and, consequently, stress-induced disorders like anxiety and depression (Anxiolytic-Like and Antidepressant-Like Activities of MCL0129 (1-[(S)-2-(4-Fluorophenyl)-2-(4-isopropylpiperadin-1-yl)ethyl]-4- [4-(2-methoxynaphthalen-1-yl)butyl]piperazine), a Novel and Potent Nonpeptide Antagonist of the Melanocortin-4 Receptor; Shigeyuki Chaki et al, J. Pharm. Exp. Ther. (2003)304(2), 818-26).

Chronic diseases such as malignant tumors or infections are frequently associated with cachexia resulting from a combination of a decrease in appetite and a loss of lean body mass. Extensive loss of lean body mass is often triggered by an inflammatory process and is usually associated with increased plasma levels of cytokines (e.g. TNF-α), which increase the production of α-MSH in the brain. Activation of MC4 receptors in the hypothalamus by α-MSH reduces appetite and increases energy expenditure. Experimental evidence in tumor bearing mice suggests that cachexia can be prevented or reversed by genetic MC4 receptor knockout or MC4 receptor blockade. The increased body weight in the treated mice is attributable to a larger amount of lean body mass, which mainly consists of skeletal muscle (Marks D.L. *et al*. Role of the central melanocortin system in cachexia. Cancer Res. (2001) 61: 1432-1438).

WO03009847A1 and WO03009850A1 describe the use of phenylpiperazinyl-phenylalanine derivatives for the treatment of obesity. Most of the compounds in both patents contain a N-(2-piperidin-4-yl-phenyl)-alkyl, benzyl or aryl sulfonamide group and N-(2-piperazin-4-yl-phenyl)-alkyl, benzyl or aryl sulfonamide group, respectively. In WO030009847A1 four out of 429 described examples bear the 1-(2-piperidin-4-yl-benzyl)-pyrrolidin-2-one-4-yl group, in WO030009850A1 four out of 456 described examples bear the 1-(2-piperazin-1-yl-benzyl)-pyrrolidin-2-one-4-yl group. However, neither the synthesis of the intermediate 1-(2-piperidin-4-yl-benzyl)-pyrrolidin-2-one nor the synthesis of the intermediate 1-(2-piperazin-1-yl-benzyl)-pyrrolidin-2-one nor the corresponding final products are described. All of the eight compounds have in common the p-chlorophenylalanine moiety which is acylated with unsubstituted and substituted azetidine-3-carboxylic acids. Other amino acids were not used to acylate the p-chlorophenylalanine. Biological data (e.g. binding IC₅₀ or functional activity) are not provided.

In view of the unresolved deficiencies in treatment of various diseases and disorders as discussed above, it is an object of the present invention to make a synthetic route for the new building blocks 1-(2-piperidin-4-yl-benzyl)-pyrrolidin-2-one and 1-(2-piperazin-4-yl-benzyl)-pyrrolidin-2-one available. These building blocks are used to provide novel substituted piperidine and piperazine derivatives with enhanced activity and improved ability to cross the blood brain barrier, which are useful as melanocortin-4 receptor agonists to treat obesity, diabetes, sexual dysfunction, and other diseases with MC-4R involvement.

### Summary of the Invention

The present invention relates to novel substituted N-benzyl-lactam derivatives of the following general structural formula.

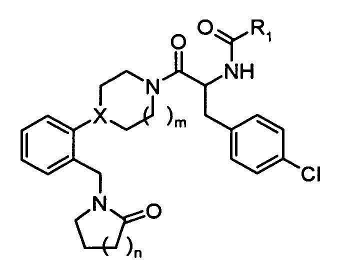

These N-benzyl-lactam derivatives are effective as melanocortin receptor agonists and are particularly effective as selective melanocortin-4 receptor (MC-4R) agonists. They are therefore useful for the treatment of disorders where the activation of the MC-4R are involved. Agonists can be used for the treatment of disorders and diseases such as obesity, diabetes, and sexual dysfunction.

The present invention also relates to pharmaceutical compositions comprising the compounds of the present invention and a pharmaceutically acceptable carrier.

### Detailed Description of the Invention

The present invention relates to novel substituted N-benzyl-lactam derivatives useful as melanocortin receptor modulators, in particular, selective MC-4R agonists.

In one embodiment, the compounds of the present invention are represented by structural formula (I). 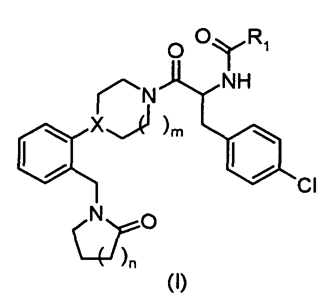 or a pharmaceutically acceptable salt or solvate thereof, wherein
X is N or CH;
R₁ is: 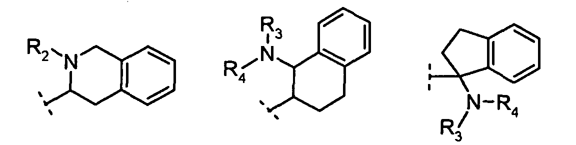 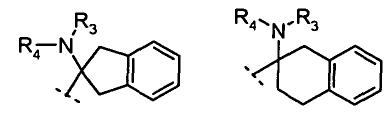 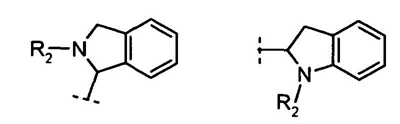 R₂ is:
hydrogen,
alkyl, or
cycloalkyl;
R₃ and R₄ are each independently:
hydrogen,
alkyl,
cycloalkyl, or
R₃ and R₄ together with the nitrogen to which they are attached form a 5- to 8-membered ring;
n = 1, 2, 3;
m=1,2.

In preferred embodiments, the variants of formula (I) have the following meanings:
R₁ is as defined above, preferably

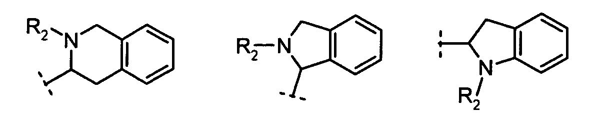

In one embodiment, R₁ is preferably

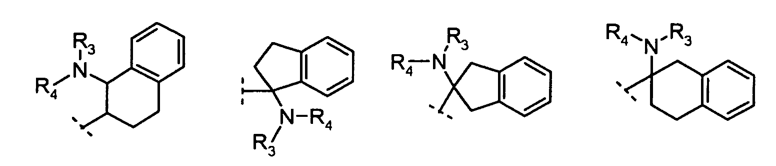

It is particularly preferred that R₁ is 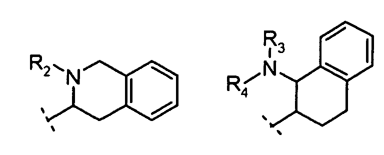 R₂ is hydrogen or alkyl, more preferably hydrogen;
each R₃ and R₄ are independently hydrogen, alkyl or cycloalkyl, more preferably hydrogen or alkyl, most preferably hydrogen;
n = 1, 2, more preferably n = 1;
m = 1
X is as defined above. In one embodiment, X is CH.

Preferred compounds in accordance with the present invention include

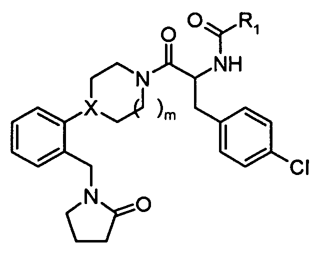

Preferred compounds of the present invention also include

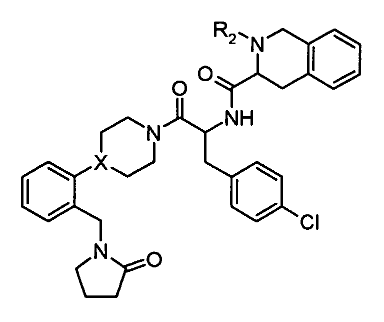

Preferred compounds of the present invention include further

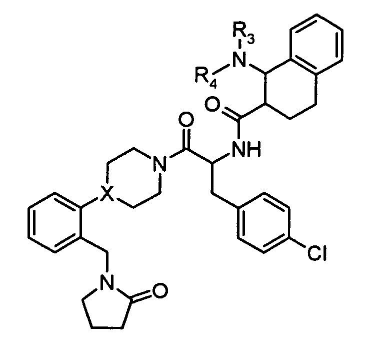

Preferred compounds of the present invention include further

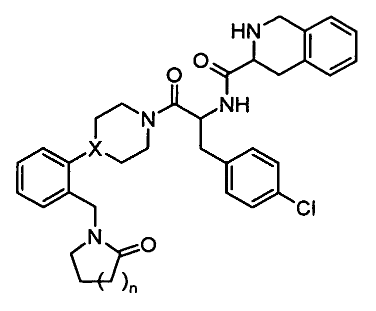

Preferred compounds of the present invention can be represented by the following formula

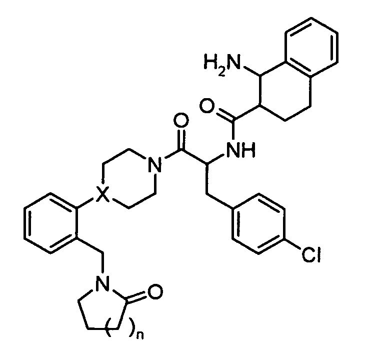

In the above, any of the preferred definitions for each variant can be combined with the preferred definition of the other variants.

In the above and the following, the employed terms have the meaning as described below:

Alkyl is straight chain or branched alkyl having preferably 1 to 8 carbon atoms, such as methyl, ethyl, n-propyl, isopropyl, n-butyl, isobutyl, tert-butyl, n-pentyl, isopentyl, neopentyl, hexyl or heptyl, more preferably 1 to 4 carbon atoms.

Cycloalkyl is an alkyl ring having preferably 3 to 8 carbon atoms, such as cyclopropyl, cyclobutyl, cyclopentyl, cyclohexyl or cycloheptyl, more preferably 3 to 6 carbon atoms.

Alkyl and cycloalkyl can be substituted or unsubstituted. If alkyl and cycloalkyl are substituted, they contain preferably one or two substituents. Examples of the substituents include F, hydroxyl, methoxy, ethoxy, isopropoxy and trifluoromethoxy. Particularly preferred substituents include F and methoxy. Preferably alkyl and cycloalkyl are unsubstituted.

The compounds of structural formula (I) are effective as melanocortin receptor agonists and are particularly effective as selective agonists of MC-4R. They are therefore useful for the treatment and/or prevention of disorders responsive to the activation of MC-4R, such as obesity, diabetes, sexual dysfunction and other diseases with MC-4R involvement.

### Optical Isomers - Diastereomers - Geometric Isomers - Tautomers

The compounds of structural formula (I) contain one or more asymmetric centers and can occur as racemates and racemic mixtures, single enantiomers, diastereomeric mixtures and individual diastereomers. The present invention is meant to comprehend all such isomeric forms of the compounds of structural formula (I).

Some of the compounds described herein may exist as tautomers such as keto-enol tautomers. The individual tautomers, as well as mixtures thereof, are encompassed within the compounds of structural formula (I).

The compounds of structural formula (I) may be separated into their individual diastereoisomers by, for example, fractional crystallization from a suitable solvent, for example, methanol or ethyl acetate or a mixture thereof, or via chiral chromatography using an optically active stationary phase. Absolute stereochemistry may be determined by X-ray crystallography of crystalline products or crystalline intermediates which are derivatized, if necessary, with a reagent containing an asymmetric center of known absolute configuration.
Alternatively, any stereoisomer of a compound of the general formula (I) may be obtained by stereospecific synthesis using optically pure starting materials or reagents of known absolute configuration.

### Salts

The term "pharmaceutically acceptable salts" refers to salts prepared from pharmaceutically acceptable non-toxic bases or acids including inorganic or organic bases and inorganic or organic acids. Salts derived from inorganic bases include aluminum, ammonium, calcium, copper, ferric, ferrous, lithium, magnesium, manganic, manganous, potassium, sodium, zinc, salts and the like. Particularly preferred are the ammonium, calcium, lithium, magnesium, potassium and sodium salts. Salts derived from pharmaceutically acceptable organic non-toxic bases include salts of primary, secondary and tertiary amines, substituted amines including naturally occurring substituted amines, cyclic amines and basic ion exchange resins such as arginine, betaine, caffeine, choline, N,N'-dibenzylethylenediamine, diethylamine, 2-diethylaminoethanol, 2-dimethylaminoethanol, ethanolamine, ethylenediamine, N-ethylmorpholine, N-ethylpiperidine, glucamine, glucosamine, histidine, hydrabamine, isopropylamine, lysine, methylglucamine, morpholine, piperazine, piperidine, polyamine resins, procaine, purines, theobromine, triethylamine, trimethylamine, tripropylamine, tromethamine and the like.

When the compound of the present invention is basic, salts may be prepared from pharmaceutically acceptable non-toxic acids, including inorganic and organic acids. Such acids include acetic, benzenesulfonic, benzoic, camphorsulfonic, citric, ethanesulfonic, formic, fumaric, gluconic, glutamic, hydrobromic, hydrochloric, isethionic, lactic, maleic, malic, mandelic, methanesulfonic, malonic, mucic, nitric, parnoic, pantothenic, phosphoric, propionic, succinic, sulfuric, tartaric, ptoluenesulfonic, trifluoroacetic acid and the like. Particularly preferred are citric, fumaric, hydrobromic, hydrochloric, maleic, phosphoric, sulfuric and tartaric acids.

It will be understood that, as used herein, references to the compounds of formula (I) are meant to also include the pharmaceutically acceptable salts.

### Utility

The compounds of formula (I) are melanocortin receptor agonists and, as such, are useful in the treatment, control or prevention of diseases, disorders or conditions responsive to the activation of one or more of the melanocortin receptors including, but not limited to, MC-1 R, MC-2R, MC-3R, MC-4R and MC-5R.

The compounds encompassed by formula (I) show highly selective affinity for the melanocortin-4 receptor relative to MC-1 R, MC-2R, MC-3R and MC-5R, which makes them especially useful in the prevention and treatment obesity, as well as male and/or female sexual dysfunction, including erectile dysfunction. "Male sexual dysfunction" includes impotence, loss of libido and erectile dysfunction. "Female sexual dysfunction" can be seen as resulting from multiple components, including dysfunction in desire, sexual arousal, sexual receptivity and orgasm.

### Administration and Dose Ranges

Any suitable route of administration may be employed for providing a mammal, especially a human with an effective dosage of a compound of the present invention. For example, oral, rectal, topical, parenteral, ocular, pulmonary, nasal and the like may be employed. Dosage forms include tablets, troches, dispersions, suspensions, solutions, capsules, creams, ointments, aerosols and the like. Preferably the compounds of formula (I) are administered orally or topically.
The effective dosage of active ingredient employed may vary depending on the particular compound employed, the mode of administration, the condition being treated and the severity of the condition being treated. Such dosage may be ascertained readily by a person skilled in the art.

When treating obesity, in conjunction with diabetes and/or hyperglycemia, or alone, generally satisfactory results are obtained when the compounds of the present invention are administered at a daily dosage of from about 0.001 milligram to about 100 milligrams per kilogram of body weight, preferably given in a single dose or in divided doses two to six times a day, or in sustained release form. In the case of a 70 kg adult human, the total daily dose will generally be from about 0.07 milligrams to about 3500 milligrams. This dosage regimen may be adjusted to provide the optimal therapeutic response.

When treating diabetes mellitus and/or hyperglycemia as well as other diseases or disorders for which the compounds of formula (I) are useful, generally satisfactory results are obtained when the compounds of the present invention are administered at a daily dosage of from about 0.001 milligram to about 100 milligram per kilogram of animal body weight, preferably given in a single dose or in divided doses two to six times a day, or in sustained release form. In the case of a 70 kg adult human, the total daily dose will generally be from about 0.07 milligrams to about 3500 milligrams. This dosage regimen may be adjusted to provide the optimal therapeutic response.

For the treatment of sexual dysfunction, the compounds of the present invention are given in a dose range of 0.001 milligram to about 100 milligram per kilogram of body weight, preferably as a single dose orally, or as a nasal spray.

### Formulation

The compound of formula (I) is preferably formulated into a dosage form prior to administration. Accordingly, the present invention also includes a pharmaceutical composition comprising a compound of formula (I) and a suitable pharmaceutical carrier.

The present pharmaceutical compositions are prepared by known procedures using well-known and readily available ingredients. In making the compositions of the present invention, the active ingredient (a compound of formula (I)) is usually mixed with a carrier or diluted by a carrier or enclosed within a carrier, which may be in the form of a capsule, sachet, paper or other container. When the carrier serves as a diluent, it may be a solid, semisolid, or liquid material which acts as a vehicle, excipient or medium for the active ingredient. Thus, the compositions can be in the form of tablets, pills, powders, lozenges, sachets, cachets, elixirs, suspensions, emulsions, solutions, syrups, aerosol (as a solid or in a liquid medium), soft and hard gelatin capsules, suppositories, sterile injectable solutions or sterile packaged powders.

Some examples of suitable carriers, excipients and diluents include lactose, dextrose, sucrose, sorbitol, mannitol, starches, gum acacia, calcium phosphate, alginates, tragacanth, gelatin, calcium silicate, microcrystalline cellulose, polyvinylpyrrolidone, cellulose, water syrup, methyl cellulose, methyl and propylhydroxybenzoates, talc, magnesium stearate and mineral oil. The formulations can additionally include lubricating agents, wetting agents, emulsifying and suspending agents, preserving agents, sweetening agents or flavoring agents. The compositions of the invention may be formulated so as to provide quick, sustained or delayed release of the active ingredient after administration to the patient.

### Preparation of Compounds of the Invention

When describing the preparation of the compounds of formula (I), the terms "A moiety", "B moiety", and "C moiety" are used below. This moiety concept is illustrated below:

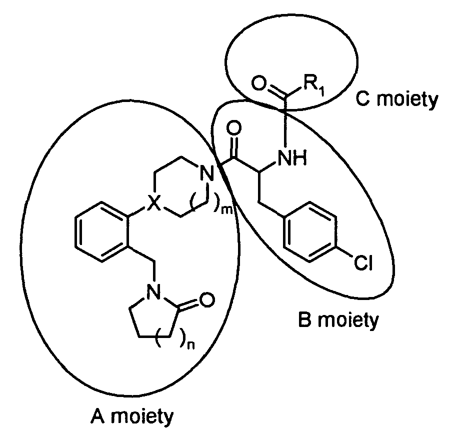

The preparation of the compounds of the present invention may be carried out via sequential or convergent synthetic routes. The skilled artisan will recognize that, in general, the three moieties of a compound of formula (I) are connected via amide bonds. The skilled artisan can, therefore, readily envision numerous routes and methods of connecting the three moieties via standard peptide coupling reaction conditions.

The phrase "standard peptide coupling reaction conditions" means coupling a carboxylic acid with an amine using an acid activating agent such as EDC, dicyclohexylcarbodiimide or benzotriazol-1-yloxytris(dimethylamino)-phosphonium hexafluorophosphate, in an inert solvent such as DCM, in the presence of a catalyst such as HOBt. The uses of protective groups for amine and carboxylic acids to facilitate the desired reaction and minimize undesired reactions are well documented. Conditions required to remove protecting groups which may be present can be found in Greene et al., Protective Groups in Organic Synthesis, John Wiley & Sons, Inc., New York, NY 1991.

Protecting groups like Z, Boc or Fmoc are used extensively in the synthesis, and their removal conditions are well known to those skilled in the art. For example, removal of Z groups can be achieved by catalytic hydrogenation with hydrogen in the presence of a noble metal or its oxide, such as palladium on activated carbon in a protic solvent such as ethanol. In cases where catalytic hydrogenation is contraindicated by the presence of other potentially reactive functionality, removal of Z can also be achieved by treatment with a solution of hydrogen bromide in acetic acid or by treatment with a mixture of TFA and dimethylsulfide. Removal of Boc protecting groups is carried out in a solvent, such as methylene chloride, methanol or ethyl acetate, with a strong acid, such as TFA, HCl or hydrogen chloride gas.

The compounds of formula (I), when existing as a diastereomeric mixture, may be separated into diastereomeric pairs of enantiomers by fractional crystallization from a suitable solvent such as methanol, ethyl acetate or a mixture thereof. The pair of enantiomers, thus obtained, may be separated into individual stereoisomers by conventional means using an optically active acid as a resolving agent. Alternatively, any enantiomer of a compound of formula (I) may be obtained by stereospecific synthesis using optically pure starting materials or reagents of known configuration.

The compounds of formula (I) of the present invention can be prepared according to the procedures of the following schemes and examples using appropriate materials and are further exemplified by the following specific examples. Moreover, by utilizing the procedures described herein, in conjunction with ordinary skills in the art, additional compounds of the present invention can be readily prepared. The compounds illustrated in the examples are not, however, to be construed as forming the only genus that is considered as the invention. The examples further illustrate details for the preparation of the compounds of the present invention. Those skilled in the art will readily understand that known variations of the conditions and processes of the following preparative procedures can be used to prepare these compounds. The instant compounds are generally isolated in the form of their pharmaceutically acceptable salts, such as those described previously. The free amine bases corresponding to the isolated salts can be generated by neutralization with a suitable base, such as aqueous sodium hydrogencarbonate, sodium carbonate, sodium hydroxide or potassium hydroxide, and extraction of the liberated amine free base into an organic solvent, followed by evaporation. The amine free base, isolated in this manner, can be further converted into another pharmaceutically acceptable salt by dissolution in an organic solvent, followed by addition of the appropriate acid and subsequent evaporation, precipitation or crystallization. All temperatures are degrees Celsius. Mass spectra (MS) were measured by electron-spray ion-mass spectroscopy.

In the schemes, preparations and examples below, the various reagent symbols and abbreviations have the following meanings:
- BINAP: 2,2'-Bis(diphenylphosphino)-1,1'-binaphtyl
- Boc: t-butoxycarbonyl
- Bz₂O₂: dibenzoylperoxide
- DCM: dichloromethane
- DIPEA: diisopropylethylamine
- DMAP: 4-dimethylaminopyridine
- DME: dimethoxyethane
- DMF: N,N-dimethylformamide
- EDC: 1-(3-dimethylaminopropyl)-3-ethylcarbodiimide hydrochloride
- Et: ethyl
- EtOAc: ethyl acetate
- Fmoc: 9-fluorenylmethyl-carbamate
- HOAc: acetic acid
- HOAt: 1-hydroxy-7-azabenzotriazole
- HOBt: 1-hydroxybenzotriazole
- h: hour(s)
- NBS: N-bromosuccinimide
- NMM: N-methylmorpholine
- Me: methyl
- Ms: methanesulfonyl
- Pd₂(dba)₃: tris(dibenzylideneacetone) dipalladium(0)
- Phe: phenylalanine
- TFA: trifluoroacetic acid
- TEA: triethylamine
- Tic: 1,2,3,4-tetrahydroisoquinoline-3-carboxylic acid
- TMOF: trimethylorthoformate
- Z: benzyloxycarbonyl

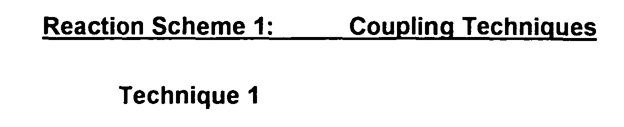

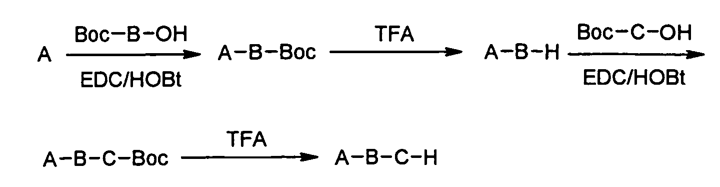

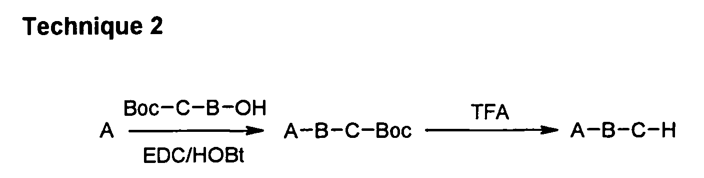

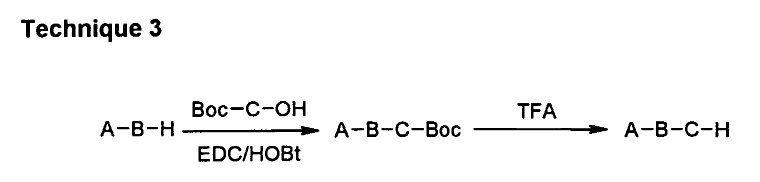

In coupling technique 1, an appropriate "A moiety" (e.g., 1-(2-piperazin-1-yl-benzyl)-pyrrolidin-2-one) is coupled to "B moiety" (e.g., D-Boc-p-Cl-Phe-OH) in the presence of EDC/HOBt followed by Boc deprotection. The coupled AB compound is then coupled to an appropriate "C moiety", followed by deprotection of Boc group and salt formation. Alternatively, when "C moiety" is not protected with Boc group, the final compound can be obtained without the deprotection step.

In coupling technique 2, an appropriate "AB moiety" is coupled to an appropriate "C moiety" in the presence of EDC/HOBt, followed by deprotection of Boc group and salt formation. Alternatively, when "C moiety" is not protected with Boc group, the final compound can be obtained without the deprotection step.
In coupling technique 3, an appropriate "BC moiety" is coupled to an appropriate "A moiety" in the presence of EDC/HOBt, followed by deprotection of Boc group and salt formation. Alternatively, when "C moiety" is not protected with Boc group, the final compound can be obtained without the deprotection step.

For coupling of A with Boc-B-OH, EDC/HOAt, EDC/HOBt or DCC/HOBt can be used.

Generally, the starting material of Boc-protected piperazine or piperidine (A moiety) can be deprotected in the presence of TFA/CH₂Cl₂, HCl/EtOAc, HCl/dioxane or HCl in MeOH/Et₂O, with or without a cation scavenger, such as dimethyl sulfide (DMS), before being subjected to the coupling procedure. It can be free-based before being subjected to the coupling procedure or, in some cases, used as the salt.

A suitable solvent such as CH₂Cl₂, DMF, THF or a mixture of the above solvents, can be used for the coupling procedure. A suitable base includes triethylamine (TEA), diisopropylethylamine (DIPEA), N-methylmorpholine (NMM), collidine and 2,6-lutidine. A base may not be needed when EDC/HOBt is used.

Generally after the reaction is completed, the reaction mixture can be diluted with an appropriate organic solvent, such as EtOAc, CH₂Cl₂ or Et₂O, which is then washed with aqueous solutions, such as water, HCl, NaHSO₄, bicarbonate, NaH₂PO₄, phosphate buffer (pH 7), brine or any combination thereof. The reaction mixture can be concentrated and then be partitioned between an appropriate organic solvent and an aqueous solution. The reaction mixture can be concentrated and subjected to chromatography without aqueous workup.

Protecting groups such as Boc, Z, Fmoc or CF₃CO, can be deprotected in the presence of H₂/Pd-C, TFA/DCM, HCl/EtOAc, HCl/dioxane, HCl in MeOH/Et₂O, NH₃/MeOH or TBAF with or without a cation scavenger, such as thioanisole, ethane thiol or dimethyl sulfide (DMS). The deprotected amines can be used as the resulting salt or are free-based by dissolving in DCM and washing with aqueous bicarbonate or aqueous NaOH. The deprotected amines can also be free-based by ion exchange chromatography.

### Reaction Schemes for Preparation of "A moiety"

The "A moieties" of the present invention, in general, may be prepared from commercially available starting materials via known chemical transformations. The preparation of "A moiety" of the compound of the present invention is illustrated in the reaction scheme below.

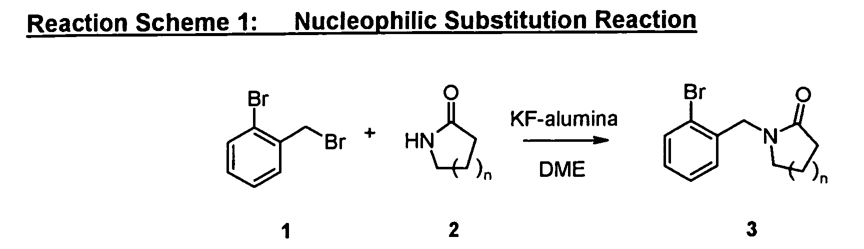

As shown in Reaction Scheme 1, the precursor for the preparation of the "A moiety" of the compounds of the present invention can be prepared by reaction with 2-bromobenzyl bromide with a lactame such as 2-pyrrolidinone in the presence of a promoter such as KF-alumina in a suitable solvent such as DME at appropriate temperature.

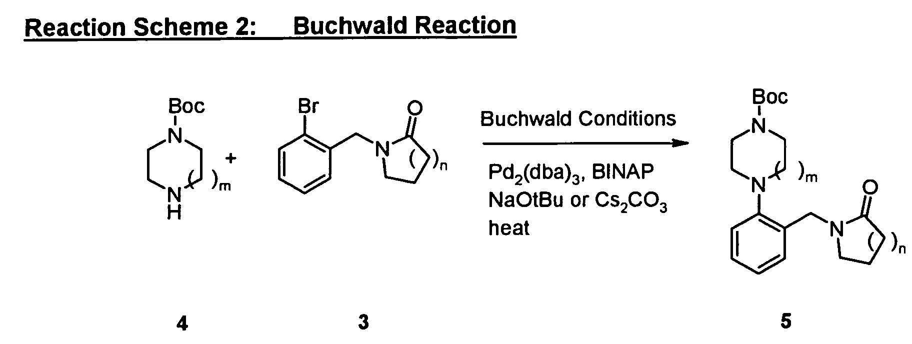

As shown in Reaction Scheme 2, the "A moiety" of the compounds of the present invention can be prepared by coupling halo-substituted aryl 3 with mono-Boc-protected diamines 4 in the presence of tri(dibenzylideneacetone) dipalladium (Pd₂(dba)₃), 2,2'-bis(diphenylphosphino)-1,1'-binaphtyl (BINAP), and sodium-tert-butoxide (NaOtBu) or cesium carbonate (Cs₂CO₃) in an organic solvent, such as toluene, at a suitable temperature. More detailed examples of "A moiety" preparation are described below.

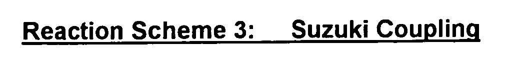

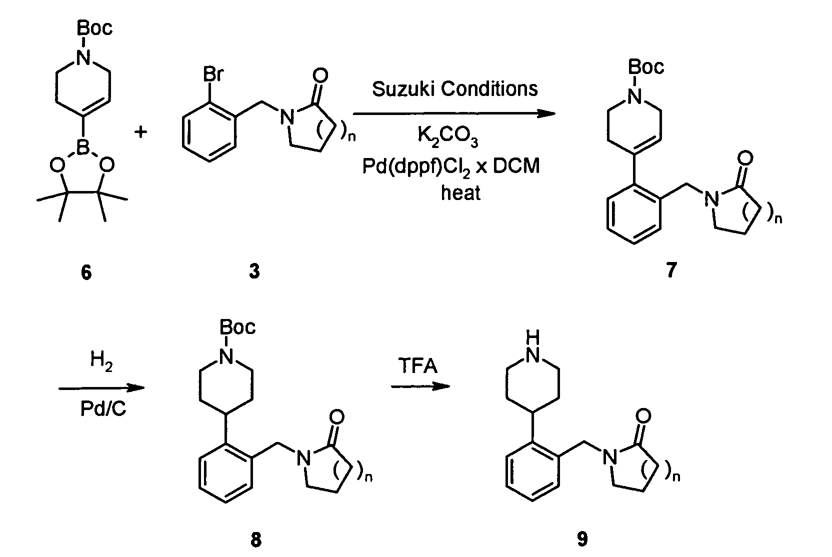

As shown in Reaction Scheme 3, 1-(2(H)-pyridine-carboxylic acid-3,6-dihydro-4-(4,4,5,5-tetramethyl-1,3,2-dioxaborolan-2-yl)-1,1-dimethyl ethyl ester **6** *(Tetrahedron Lett*. 2000, 41, 3705-3708) can be reacted with haloaromates such as **3** in the presence of a base such as K₂CO₃ and a catalyst such as dichloro(1,1'-bis(diphenylphosphino)-ferrocene)palladium(II) DCM adduct in an organic solvent such as DMF at a suitable temperature. The tetrahydropyridines can be hydrogenated in the presence of a catalyst such as Pd/C to yield the protected piperidines **8** which can subsequently be deprotected with a reagent such as TFA to yield piperidines **9.**

1-tert-Butoxycarbonylamino-1,2,3,4-tetrahydro-naphthalene-2-carboxylic acid was prepared as described in W00191752.

The following describes the detailed examples of the invention

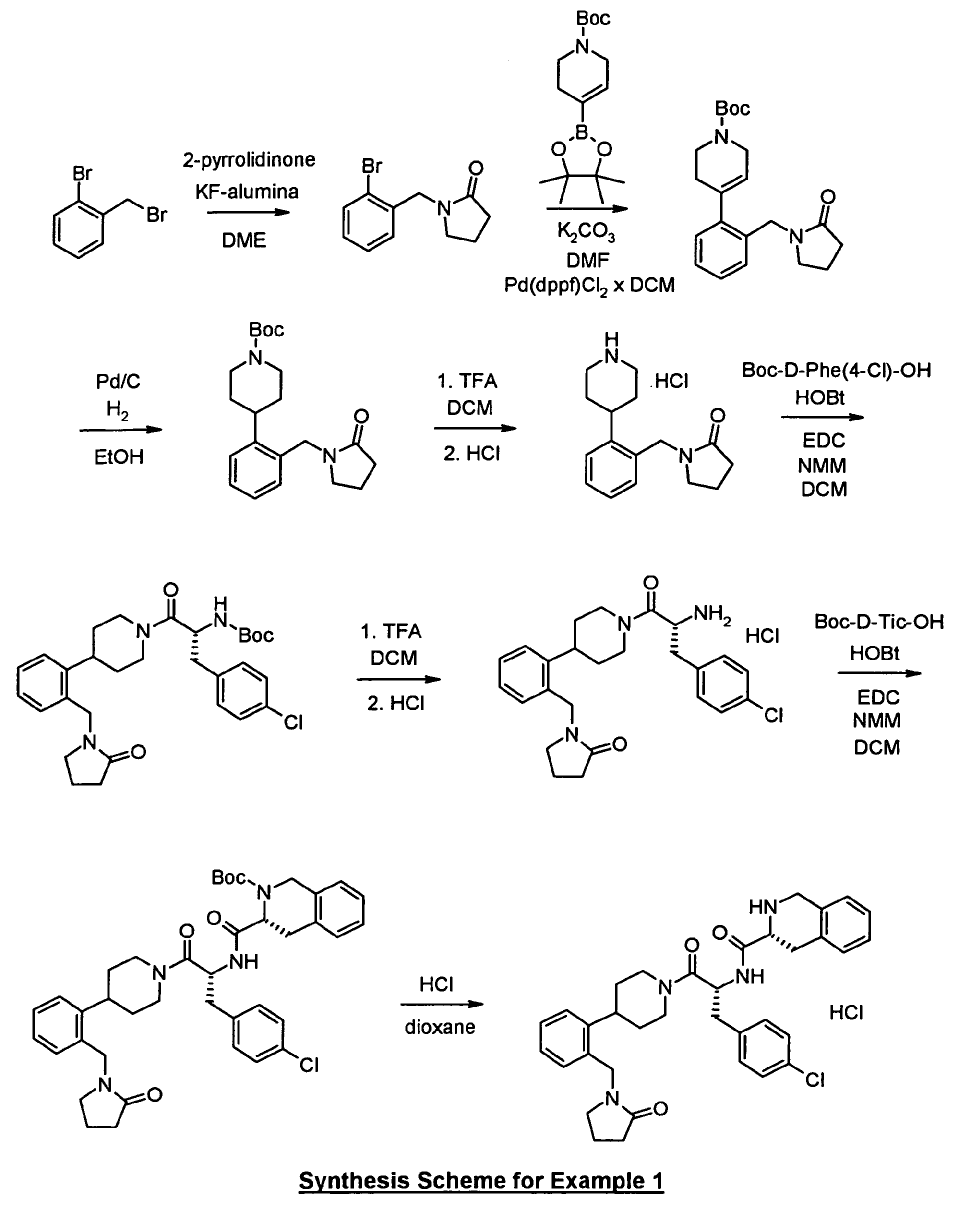

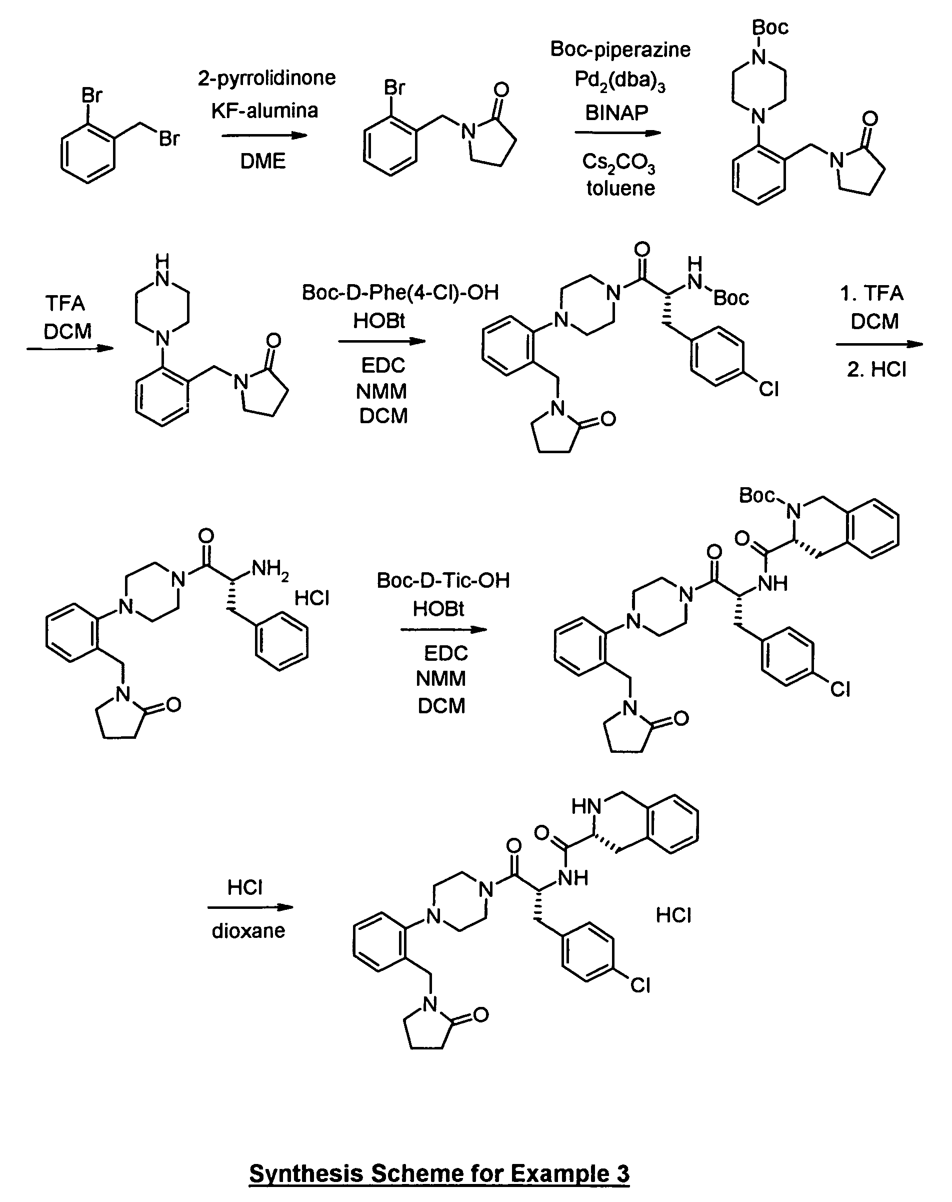

The following examples are provided to illustrate the invention and are not limiting the scope of the invention in any manner. 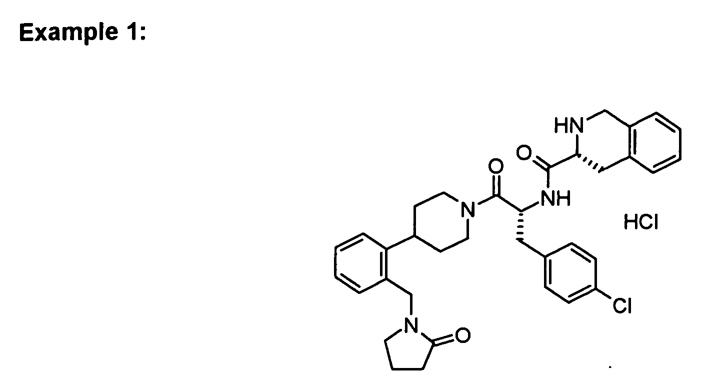 white solid

R_{f} = 0.53 (DCM/MeOH 9:1); Mp. 175 - 200 °C.

To Boc-protected intermediate 1 g) (32 mg) was added hydrogen chloride, 4.0 M sol. in 1,4-dioxane (10 ml) and the solution was stirred for 90 min at room temperature. The solvent was removed under reduced pressure. The residue was dissolved in DCM and treated with diethyl ether. The precipitate was filtered off to yield the title compound as a solid.
The required intermediates can be synthesized in the following way:

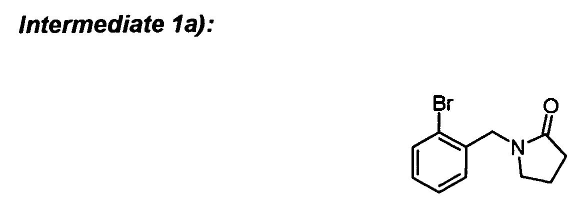

To a solution of 2-bromobenzyl bromide (3.05 g) and 2-pyrrolidinone (0.85 g) in DME (20 ml) was added KF-alumina (0.45 g) and the mixture was stirred for 48 h at room temperature. The inorganics were filtered off and the solvent was removed to afford the desired compound.

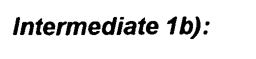

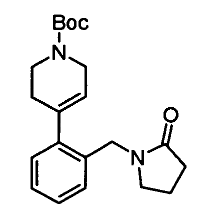

To intermediate 1a) (623 mg) in DMF (20 ml) was added 1-(2(H)-pyridine-carboxylic acid-3,6-dihydro-4-(4,4,5,5-tetramethyl-1,3,2-dioxaborolan-2-yl)-1,1-dimethyl ethyl ester (909 mg), dichloro(1,1'-bis(diphenylphosphino)-ferrocene)palladium(II) DCM adduct (108 mg) and K₂CO₃ (1002 mg). The reaction was heated to about 90°C overnight. The mixture was cooled, diluted with DCM and filtered through Celite. The filtrate was concentrated to dryness and the resulting residue was taken up in EtOAc (50 ml). The organics were washed with water, brine and concentrated to dryness. The crude product was purified by flash chromatography. 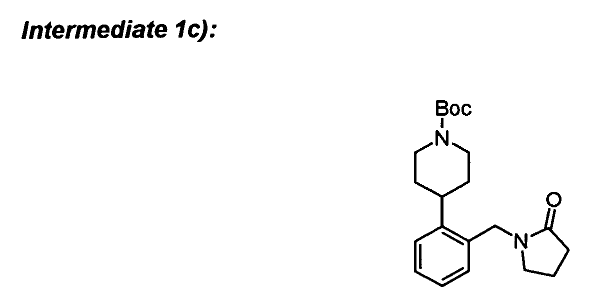 To intermediate 1b) (422 mg) in EtOH (20 ml) was added a slurry of 10% Pd/C in EtOH (20 ml). The mixture was stirred rapidly under H₂ (1 atm) for about 2 h. The reaction mixture was filtered over a pad of Celite and washed with EtOAc (100 ml). The filtrate was concentrated to dryness to yield the final compound.

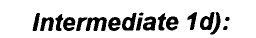

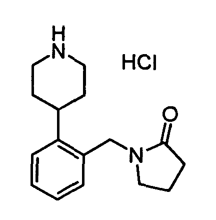

To the Boc-protected amine from 1c) (190 mg) in DCM (5 ml) was added TFA (1 ml) and stirred at room temperature for 90 min. Additional TFA (1 ml) was added and stirred for 10 min. The reaction mixture was diluted with DCM (10 ml) and carefully basified by pouring into 10% aqueous sodium carbonate solution (20 ml). The organic layer was separated and the aqueous layer was further extracted three times with DCM. The combined organics were washed with water and brine, dried over Na₂SO₄, concentrated to give a white solid.

For prolonged storage, the free base was converted into the corresponding hydrochloride. The free base was dissolved in DCM (5 ml) and app. 1 M HCl in ether (10 ml) was added. The precipitate was filtered and the residue was washed three times with ether and dried under reduced pressure to yield the desired compound.

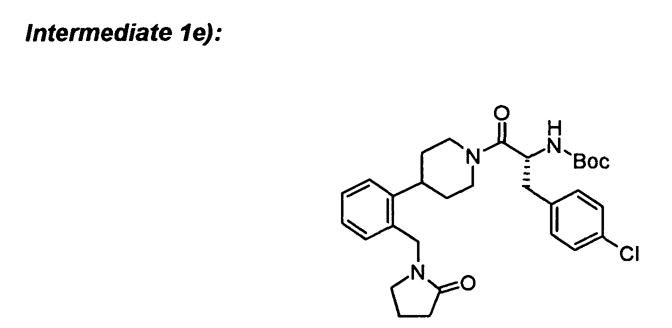

To Boc-D-4-chlorophenylalanine (82 mg) in DCM (5 ml) was added the amine hydrochloride from 1d) (42 mg), N-methylmorpholine (42 µl), HOBt (48 mg) and stirred for 20 min. EDC (72 mg) was added and stirring was continued for 1 h. An additional amount of N-methylmorpholine (20 µl) was added and stirred overnight. The reaction mixture was poured into water (5 ml) and the organic phase was separated. The aqueous phase was extracted two times with DCM. The combined organic phases were washed with 0.5 N HCl and saturated sodium bicarbonate solution, dried over Na₂SO₄ and concentrated in vacuo. Purification by column chromatography yielded the title compound.

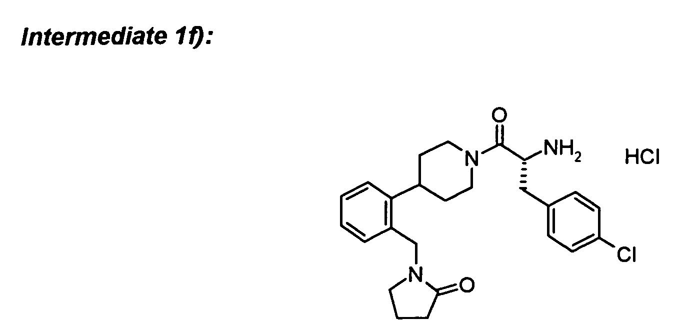

To the Boc-protected amine from 1e) (154 mg) in DCM (5 ml) was added TFA (1 ml) and stirred at room temperature for 90 min. Additional TFA (1 ml) was added and stirred for 10 min. The reaction mixture was diluted with DCM (10 ml) and carefully basified by pouring into 10% aqueous sodium carbonate solution (20 ml). The organic layer was separated and the aqueous layer was further extracted three times with DCM. The combined organics were washed with water and brine, dried over Na₂SO₄, concentrated to give a white solid.

For prolonged storage, the free base was converted into the corresponding hydrochloride. The free base was dissolved in DCM (5 ml) and app. 1 M HCl in ether (10 ml) was added. The precipitate was filtered and the residue was washed three times with ether and dried under reduced pressure to yield the desired compound.

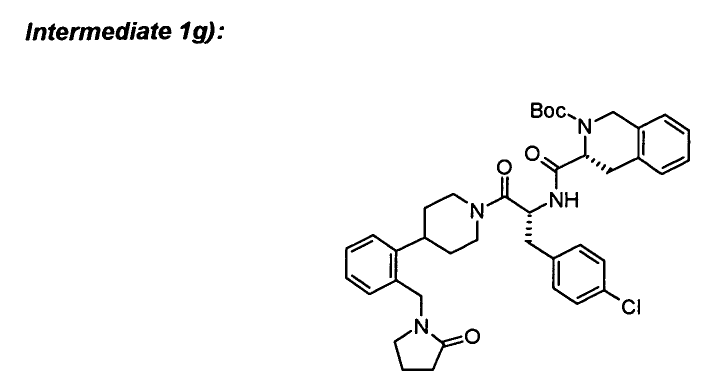

To (R)-Boc-1,2,3,4-tetrahydroisoquinoline-3-carboxylic acid (24 mg) in DCM (2 ml) was added intermediate 1f) (36 mg), N-methylmorpholine (14 µl), HOBt (14 mg) and stirred for 20 min. EDC (23 mg) was added and stirring was continued for 1 h. An additional amount of N-methylmorpholine (8 µl) was added and stirred overnight. The reaction mixture was poured into water (5 ml) and the organic phase was separated. The aqueous phase was extracted two times with ethyl acetate. The combined organic phases were washed three times with 0.5 N HCl and three times with saturated sodium bicarbonate solution, dried over Na₂SO₄ and concentrated to yield the product which was purified by column chromatography.

The following examples can be prepared in a similar way:

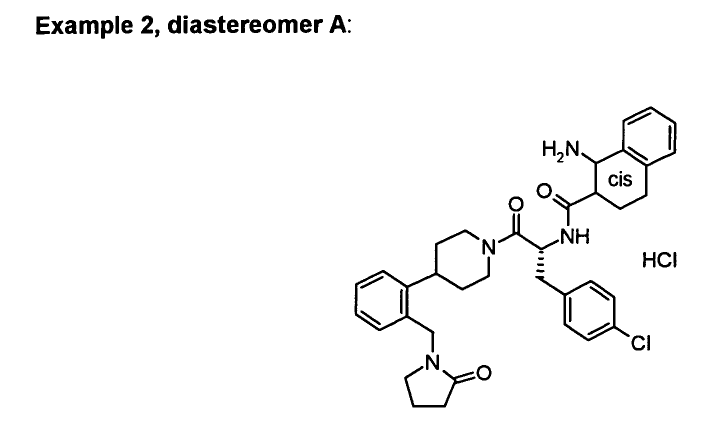

Derived from the Boc-protected precursor with higher R_{f} value.
white solid

R_{f} = 0.42 (DCM/MeOH 9:1); Mp. 180 - 200 °C.

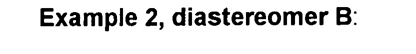

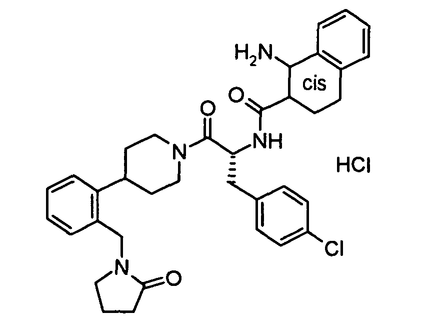

Derived from the Boc-protected precursor with lower R_{f} value.
white solid

R_{f} = 0.37 (DCM/MeOH 9:1); Mp. 185 - 205 °C.

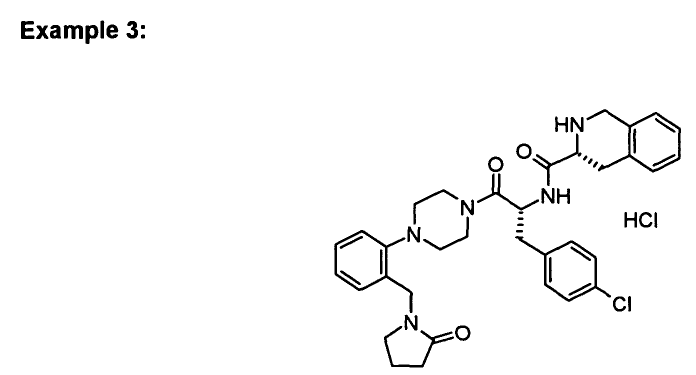

white solid
Mp. 167 - 190 °C.

To Boc-protected intermediate 3e) (31 mg) was added hydrogen chloride, 4.0 M sol. in 1,4-dioxane (10 ml) and the solution was stirred for 90 min at room temperature. The solvent was removed under reduced pressure. The residue was dissolved in DCM and treated with diethyl ether. The precipitate was filtered off to yield the title compound as a solid.

The required intermediates can be synthesized in the following way:

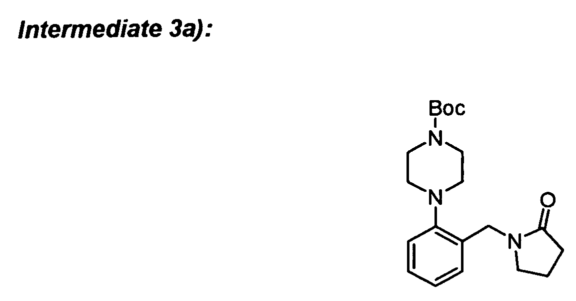

Boc-piperazine (895 mg), intermediate 1 a) (1004 mg), Pd₂(dba)₃ (235 mg), BINAP (442 mg) and cesium carbonate (3 g) were mixed together in toluene (20 ml). The mixture was degassed and heated to 100°C for 3 d. The mixture was diluted with ether (100 ml) and filtered over Celite. The filtrate was concentrated and then subjected to chromatography on silica gel to yield the title compound.

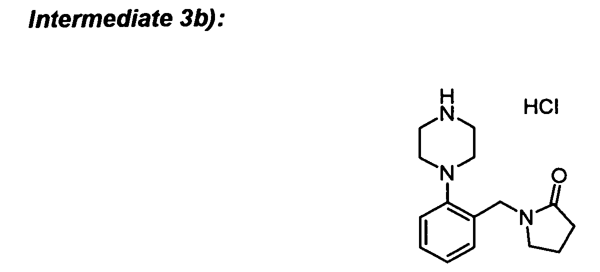

To the Boc-protected amine from 3a) (680 mg) in DCM (10 ml) was added TFA (2 ml) and stirred at room temperature for 90 min. Additional TFA (2 ml) was added and stirred for 10 min. The reaction mixture was diluted with DCM (20 ml) and carefully basified by pouring into 10% aqueous sodium carbonate solution (40 ml). The organic layer was separated and the aqueous layer was further extracted three times with DCM. The combined organics were washed with water and brine, dried over Na₂SO₄, concentrated to give a white solid.

For prolonged storage, the free base was converted into the corresponding hydrochloride. The free base was dissolved in DCM (10 ml) and app. 1 M HCl in ether (20 ml) was added.

The precipitate was filtered and the residue was washed three times with ether and dried under reduced pressure to yield the desired product.

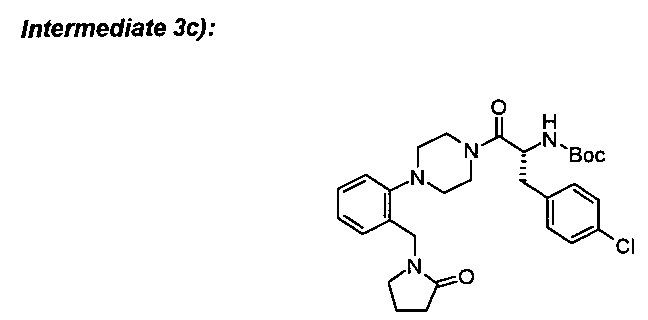

To Boc-D-4-chlorophenylalanine (82 mg) in DCM (5 ml) was added the amine hydrochloride from 3b) (61 mg), N-methylmorpholine (42 µl), HOBt (48 mg) and stirred for 20 min. EDC (72 mg) was added and stirring was continued for 1 h. An additional amount of N-methylmorpholine (20 µl) was added and stirred overnight. The reaction mixture was poured into water (5 ml) and the organic phase was separated. The aqueous phase was extracted two times with DCM. The combined organic phases were washed with 0.5 N HCl and saturated sodium bicarbonate solution, dried over Na₂SO₄ and concentrated to yield the title compound which was purified by column chromatography.

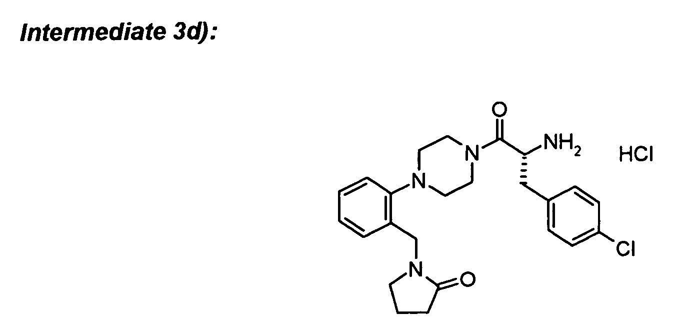

To the Boc-protected amine from 3c) (78 mg) in DCM (5 ml) was added TFA (1 ml) and stirred at room temperature for 90 min. Additional TFA (1 ml) was added and stirred for 10 min. The reaction mixture was diluted with DCM (10 ml) and carefully basified by pouring into 10% aqueous sodium carbonate solution (20 ml). The organic layer was separated and the aqueous layer was further extracted three times with DCM. The combined organics were washed with water and brine, dried over Na₂SO₄, concentrated to give a white solid.

For prolonged storage, the free base was converted into the corresponding hydrochloride. The free base was dissolved in DCM (5 ml) and app. 1 M HCl in ether (10 ml), was added. The precipitate was filtered and the residue was washed three times with ether and dried under reduced pressure to yield the desired product.

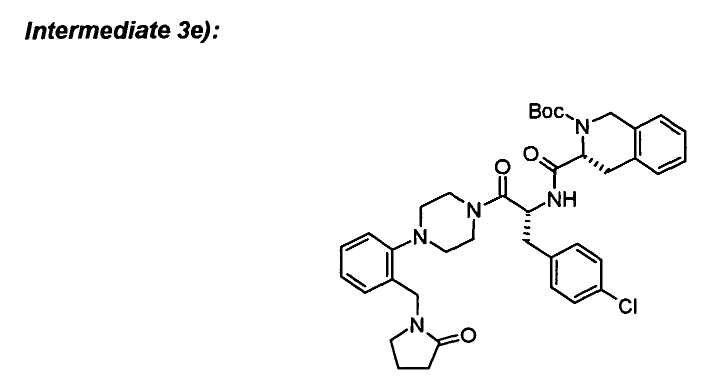

To (R)-Boc-1,2,3,4-tetrahydroisoquinoline-3-carboxylic acid (24 mg) in DCM (2 ml) was added intermediate 3d) (36 mg), N-methylmorpholine (14 µl), HOBt (14 mg) and stirred for 20 min. EDC (23 mg) was added and stirring was continued for 1 h. An additional amount of N-methylmorpholine (8 µl) was added and stirred overnight. The reaction mixture was poured into water (5 ml) and the organic phase was separated. The aqueous phase was extracted two times with ethyl acetate. The combined organic phases were washed three times with 0.5 N HCl and three times with saturated sodium bicarbonate solution, dried over Na₂SO₄ and concentrated to yield the product which was purified by column chromatography.

The following examples can be prepared in a similar way:

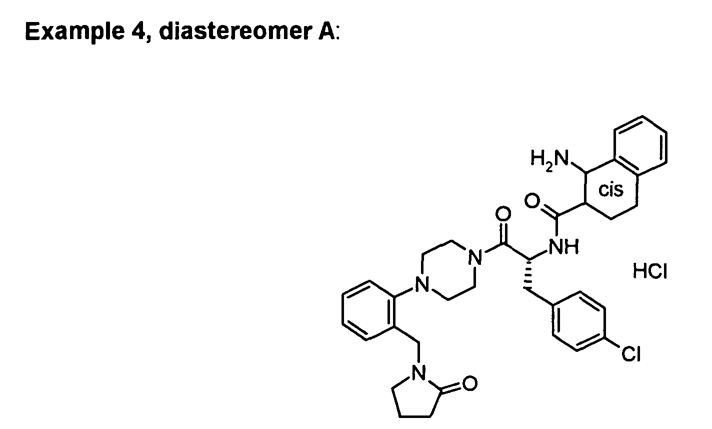

Derived from the Boc-protected precursor with higher R_{f} value.
white solid
Mp. 143 - 151 °C.

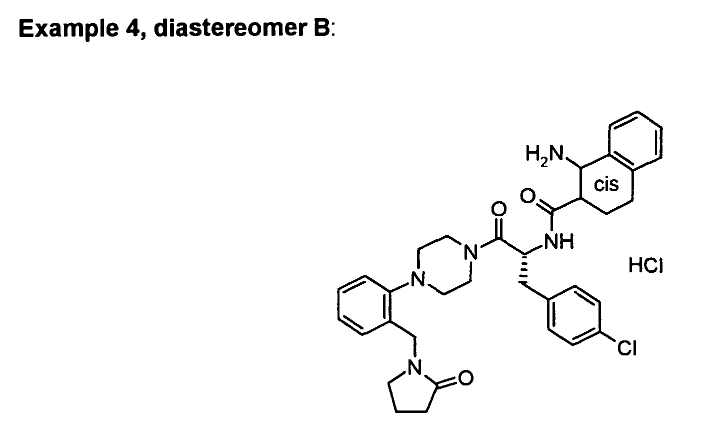

Derived from the Boc-protected precursor with lower R_{f} value.
white solid
Mp. 159 - 166 °C.

### BIOLOGICAL ASSAYS

### A. Binding Assay

A membrane binding assay is used to identify competitive inhibitors of fluorescence labeled NDP-alpha-MSH binding to HEK293 cell membrane preparations expressing human melanocortin receptors.

The test compound or unlabeled NDP-alpha-MSH is dispensed at varying concentrations to a 384 well microtiter plate. Fluorescence labeled NDP-alpha-MSH is dispensed at a single concentration, followed by addition of membrane preparations. The plate is incubated for 5 h at room temperature.

The degree of fluorescence polarization is determined with a fluorescence polarization microplate reader.

### B. Functional Assay

A functional cellular assay, based on competition between unlabeled cAMP and a fixed quantity of fluorescence labeled cAMP for a limited number of binding sites on a cAMP specific antibody, is used to discriminate melanocortin receptor agonists from antagonists by fluorescence polarization.

HEK293 cells expressing one of the human melanocortin receptors are transferred to 384 well microtiter plates, an appropriate amount of cAMP antibody is added, followed by the addition of different concentrations of the test compound to effect cAMP production. Cells are lysed and a fluorescence labeled cAMP conjugate is dispensed. The plate is read on a fluorescence polarization microplate reader and the amount of cAMP produced as a response to a test compound is compared to the production of cAMP resulting from stimulation with NDP-alpha-MSH.

To define antagonistic activity of a test compound, the compound is dispensed at different concentrations to cells stimulated by an appropriate amount of NDP-α-MSH. Inhibition of cAMP production is determined by comparing the inhibition of cAMP production of the test compound to the inhibition of cAMP production by a known inhibitor tested at the same concentrations.

In vitro data for the Examples of the present patent application:

**Table 1.**

| | Binding Activity IC50 (nM) | | |
|---|---|---|---|
| Example | hMC-3R | hMC-4R | hMC-5R |
| 1 | 7200 | 17 | 807 |
| 2A | | 17 | |
| 2B | | 7 | |
| 3 | 41%@10 uM | 29 | 1300 |
| 4B | 47%@10 uM | 21 | 1800 |

**Table 2.**

| Functional Activity EC50 (nM) | | |
|---|---|---|
| Example | hMC-4R | % activation |
| 1 | 60 | 112 |
| 2A | 101 | 86 |
| 2B | 29 | 90 |
| 3 | 260 | 109 |
| 4B | 180 | 91 |

In vitro data for selected compounds from patents WO030009847 and WO030009850:

**Table 3.**

| Binding Activity IC50 (nM) | | | | |
|---|---|---|---|---|
| | Example | hMC-3R | hMC-4R | hMC-5R |
| WO030009847 | 153 | 5200 | 40 | 3300 |
| WO030009850 | 1 | | 500 | |
| WO030009850 | 34 | | 2900 | |
| WO030009850 | 166 | 19200 | 110 | 6900 |
| WO030009850 | 169 | | 79 | |
| WO030009850 | 170 | | 83 | |

**Table 4.**

| | Functional Activity EC50 (nM) | | |
|---|---|---|---|
| | Example | hMC-4R | % activation |
| WO030009847 | 153 | 1020 | 107 |
| WO030009850 | 1 | 3740 96 | |
| WO030009850 | 34 | 11%@10 µM | |
| WO030009850 | 166 | 2850 | 90 |
| WO030009850 | 169 | 2410 | 47 |
| WO030009850 | 170 | 6310 | 46 |

The compounds of the present invention show improved potency in the binding assay and better functional activity compared with the examples of WO03009847A1 and WO03009850A1.

Piperidine linked Examples **1**, **2A** and **2B** of the present invention where azetidine-3-carboxylic acid has been replaced by other C moieties have to be compared with (R)-azetidine-3-carboxylic acid (1-(4-chloro-benzyl)-2-oxo-2-{4-[2-(2-oxo-pyrrolidin-1-ylmethyl)-phenyl]-piperidin-1-yl}-ethyl)-amide (WO030009847A1, Example 153). The compounds have hMC4 EC₅₀ values between 29 and 101 nM (see Table 2.) which means that they show 10 to 35fold better functional activity than the reference compound. The Amgen example 153 from WO030009847A1 shows an hMC4 EC₅₀ of 1020 nM (see Table 4.).
Similar observations can be made for Examples **3** and **4B** of the present invention. In these cases the functional activity is 11fold and 16fold, respectively, better than the one from (R)-azetidine-3-carboxylic acid (1-(4-chloro-benzyl)-2-oxo-2-{4-[2-(2-oxo-pyrrolidin-1-ylmethyl)-phenyl]-piperazin-1-yl}-ethyl)-amide (WO030009850A1, Example 166). However, the 1-(2-piperazin-1-yl-benzyl)-pyrrolidin-2-one-4-yl group is only used as A moiety for four examples. No synthesis procedure and no biological data were given for this novel class of compounds. Comparison of Examples **3** and **4B** of the present invention with the more representive examples (R,R)-1,2,3,4-tetrahydro-isoquinoline-3-carboxylic acid {1-(4-chloro-benzyl)-2-[4-(2-methanesulfonylamino-phenyl)-piperazin-1-yl]-2-oxo-ethyl}-amide and (R)-azetidine-3-carboxylic acid {1-(4-chloro-benzyl)-2-[4-(2-methanesulfonylamino-phenyl)-piperazin-1-yl]-2-oxo-ethyl}-amide (WO030009850A1, Examples 1 and 34) shows an even larger improvement (more than 14fold and 21fold, resp.).

### C. In Vivo Food Intake Models

### 1. Spontaneous Feeding Paradigm

Food intake in rats is measured after i.p. or p.o. administration of the test compound (see e.g. Chen, A.S. et al. Transgenic Res 2000 Apr 9(2):145-54)

### D. Rat Ex Copula Assay

Sexually mature male Caesarian Derived Sprague Dawley (CD) rats (over 60 days old) are used with the suspensory ligament surgically removed to prevent retraction of the penis, back into the penile sheath during the ex copula evaluations. Animals receive food and water ad lib and are kept on a normal light/dark cycle. Studies are conducted during the light cycle.

### 1. Conditioning to Supine Restraint for Ex Copula Reflex Tests

This conditioning takes about 4 days. Day 1, the animals are placed in a darkened restrainer and left for 15 - 30 minutes. Day 2, the animals are restrained in a supine position in the restrainer for 15 - 30 minutes. Day 3, the animals are restrained in the supine position with the penile sheath retracted for 15 - 30 minutes. Day 4, the animals are restrained in the supine position with the penile sheath retracted until penile responses are observed. Some animals require additional days of conditioning before they are completely acclimated to the procedures; non-responders are removed from further evaluation. After any handling or evaluation animals are given a treat to ensure positive reinforcement.

### 2. Ex Copula Reflex Tests

Rats are gently restrained in a supine position with their anterior torso placed inside a cylinder of adequate size to allow for normal head and paw grooming. For a 400 - 500 gram rat, the diameter of the cylinder is approximately 8 cm. The lower torso and hind limbs are restrained with a nonadhesive material (vetrap). An additional piece of vetrap with a hole in it, through which the glans penis will be passed, is fastened over the animal to maintain the preputial sheath in a retracted position. Penile responses will be observed, typically termed ex copula genital reflex tests. Typically, a series of penile erections will occur spontaneously within a few minutes after sheath retraction. The types of normal reflexogenic erectile responses include elongation, engorgement, cup and flip. An elongation is classified as an extension of the penile body. Engorgement is a dilation of the glans penis. A cup is defined as an intense erection where the distal margin of the glans penis momentarily flares open to form a cup. A flip is a dorsiflexion of the penile body.

Baseline and or vehicle evaluations are conducted to determine how and if an animal will respond. Some animals have a long duration until the first response while others are non-responders altogether. During this baseline evaluation latency to first response, number and type of responses are recorded. The testing time frame is 15 minutes after the first response.

After a minimum of 1 day between evaluations, these same animals are administered the test compound at 20 mg/kg and evaluated for penile reflexes. All evaluations are videotaped and scored later. Data are collected and analyzed using paired, 2 tailed t-tests, to compared baseline and/or vehicle evaluations to drug treated evaluations for individual animals. Groups of a minimum of 4 animals are utilized to reduce variability.

Positive reference controls are included in each study to assure the validity of the study. Animals can be dosed by a number of routes of administration depending on the nature of the study to be performed. The routes of administration includes intravenous (IV), intraperitoneal (IP), subcutaneous (SC) and intracerebral ventricular (ICV).

### E. Models of Female Sexual Dysfunction

Rodent assays, relevant to female sexual receptivity, include the behavioral model of lordosis and direct observations of copulatory activity. There is also a urethrogenital reflex model in anesthetized spinally transected rats for measuring orgasm in both male and female rats. These and other established animal models of female sexual dysfunction are described in McKenna KE et al, A Model For The Study of Sexual Function In Anesthetized Male And Female Rats, Am. J. Physiol. (Regulatory Integrative Comp. Physiol 30): R1276-R1285, 1991; McKenna KE et al, Modulation By Peripheral Serotonin of The Threshold For Sexual Reflexes In Female Rats, Pharm. Bioch. Behav., 40:151-156, 1991; and Takahashi LK et al, Dual Estradiol Action In The Diencephalon And The Regulation of Sociosexual Behavior In Female Golden Hamsters, Brain Res., 359:194-207, 1985.

Representative compounds of the present invention were tested and found to bind to the melanocortin-4 receptor. These compounds were generally found to have IC50 values less than 2 µM. Representative compounds of the present invention were also tested in the functional assay and found generally to activate the melanocortin-4 receptor with EC50 values less than 1 µM.

### Examples of a Pharmaceutical Composition

As a specific embodiment of an oral composition of a compound of the present invention, 20 mg of Example 1 is formulated with sufficient finely divided lactose to provide a total amount of 580 to 590 mg to fill a size 0 hard gelatin capsule.

As another specific embodiment of an oral composition of a compound of the present invention, 15 mg of Example 4B is formulated with sufficient finely divided lactose to provide a total amount of 580 to 590 mg to fill a size 0 hard gelatin capsule.

While the invention has been described and illustrated in reference to certain preferred embodiments thereof, those skilled in the art will appreciate that various changes, modifications and substitutions can be made therein without departing from the spirit and scope of the invention. For example, effective dosages other than the preferred doses as set forth above may be applicable as a consequence of the specific pharmacological responses observed and may vary depending upon the particular active compound selected, as well as from the type of formulation and mode of administration employed, and such expected variations or differences in the results are contemplated in accordance with the objects and practices of the present invention. It is intended, therefore, that the invention be limited only by the scope of the claims which follow and that such claims be interpreted as broadly as is reasonable.

## Claims

1. A compound of structural formula (I): 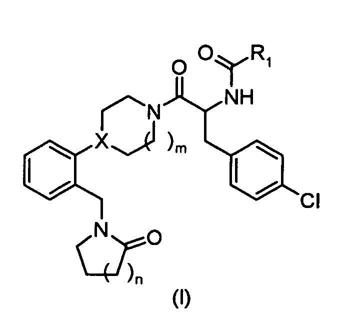 or a pharmaceutically acceptable salt or solvate thereof, wherein
X is N or CH;
R₁ is: 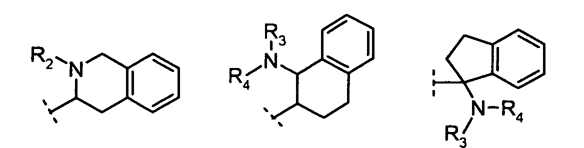 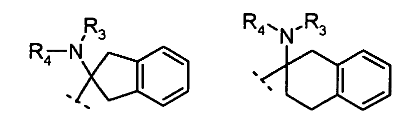 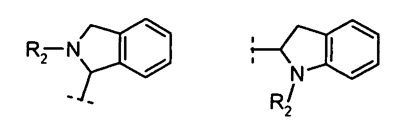
R₂ is:
hydrogen,
alkyl, or
cycloalkyl;
R₃ and R₄ are each independently:
hydrogen,
alkyl,
cycloalkyl, or
R₃ and R₄ together with the nitrogen to which they are attached form a 5- to 8-membered ring;
n = 1, 2, 3;
m = 1, 2.

2. The compound of claim 1, wherein
R₂ is hydrogen or alkyl;
each R₃ and R₄ are independently hydrogen, alkyl or cycloalkyl;
n = 1, 2;
m = 1;

3. The compound of claim 1 or 2, wherein
R₂ is hydrogen;
each R₃ and R₄ are independently hydrogen or alkyl;
n = 1;

4. The compound of any of claims 1 to 3, wherein
R₃ and R₄ are hydrogen;

5. The compound of any of claims 1 to 3 for use as a medicament.

6. Use of the compound of any of claims 1 to 3 for the preparation of a medicament for the treatment or prevention of disorders, diseases or conditions responsive to the activation of the melanocortin-4 receptor.

7. Use according to claim 5 for the treatment or prevention of obesity.

8. Use according to claim 5 for the treatment or prevention of diabetes mellitus.

9. Use according to claim 5 for the treatment or prevention of male or female sexual dysfunction.

10. Use according to claim 5 for the treatment or prevention of erectile dysfunction.

11. A pharmaceutical composition which comprises a compound of any of claims 1 to 4 and a pharmaceutically acceptable carrier.
